# EUROPEAN PATENT APPLICATION

(11) **EP 1 472 933 A1**
(43) Date of publication of application: **03.11.2004**
(21) Application number: 03076392.4
(22) Date of filing: 01.05.2003
(51) Int. Cl.: A23K 1/00, A23K 1/18, A61K 35/74

(54) **Improving health and/or nutritional status of an aquatic animal by aid of Bacillus licheniformis**

(71) Applicant: Chr. Hansen A/S, 2970 Hoersholm (DK)
(72) Inventor: Suhr-Jessen, Peter, 3500 Vaerloese (DK); Moerk Jensen, Merete, 3520 Farum (DK)

(57) **Abstract**

An aquatic animal feed product comprising a probiotic *Bacillus licheniformis* bacteria and the use of this product in an aquaculture of aquatic animals in order to improve the resistance of an aquatic animal towards influential conditions in the surrounding environment, such as e.g. pathogenic micro-organisms.

## Description

### FIELD OF THE INVENTION

The present invention relates to an aquatic animal feed product comprising a probiotic *Bacillus licheniformis* bacteria and the use of this product in an aquaculture of aquatic animals in order to improve the resistance of an aquatic animal towards influential conditions in the surrounding environment, such as e.g. pathogenic micro-organisms.

### DESCRIPTION OF THE BACKGROUND

Aquaculture has become one of the fastest-growing food-producing sectors and there has been much focus on the possibility of applying conventional methods known from livestock farming for disease control and growth promotion in aquaculture. However, the immediate ambient environment has a much larger influence on the health status of the marine animals than with terrestrial animals. Disease outbreaks caused by the presence of harmful organisms in the surrounding environment is recognized as one of the most significant constraint on aquaculture production, affecting the economic development of the sector in many countries.

Marine animals kept in cages are vulnerable towards adverse effects from the surrounding environment. Harmful pathogenic microorganisms may proliferate in the protected aquacultural environment and cause substantial problems for the farmed animals. Even microorganisms not characterized as pathogens as such but being present in huge amount may constitute a potential problem in aquacultural production.

There has been much focus on controlling presence of pathogens in the aquacultural environment by applying and testing conventional methods used in the farming of terrestrial animals. The methods include addition of antimicrobial compounds, such as e.g. antibiotics, to the environment. However, these conventional approaches have had limited success. Furthermore to the unsuccessfulness, there is a general and growing concern about the use of antimicrobials and there is a world-wide regulatory restriction on the use of antibiotics.

As expected, the need for disease control has been accounted for by the development of methods that are different from the conventional methods. One such method comprises the use of "probiotics", which are antagonistic bacterial strains to be used for the control of populations of potential pathogens through e.g. competitive exclusion. Examples of this technology are already in use within farming of terrestrial animals, where commercial probiotic products have been launched on the market. An oral administration of these products induces increased resistance to enteric infections and enhance the general immune response of the treated animal.

The prior art within aquaculture presently shows that bacterial species have been tested for control of the aquatic environment. Within aquaculture it appears from the literature, that the selected bacterial species colonize the aquatic environment including the external slime layer of fresh and salt-water aquatic animals. The effect hereof of this bacterial colonization is that harmful microorganisms are excluded and that other natural biological processes contribute to a breakdown of organic waste into carbon dioxide and water. Importantly to note is that it has additionally been found that the use of probiotics helps alleviate the harmful effects of e.g. transfer stress, which is often accompanying the transfer of one aquatic species from one environment to another. Transfer stress is featured *inter alia* by the fact that the slime layers of aquatic species lose a significant portion of their natural protective microflora. It appears that exposure to large numbers of probiotic bacteria implicates a restoration of a healthy microflora. The probiotic bacteria compete with many pathogens, such as e.g. *Aeromonas* and *Vibrio* species, which are indigenous to aquatic environments and by competitive exclusion, the frequency of disease brought about by these bacterial pathogens is significantly reduced.

WO02/00035 describes a bioactive food complex for controlling bacterial diseases in aquatic animals. The food complex is described as an emulsion comprising at least one probiotic bacteria, selected for antimicrobial chemical production, and at least one inhibitory or regulatory compound. The at least one inhibitory or regulatory compound is furanones, which comprises a group of chemical compounds that inhibit surface colonisation of microorganisms in general. As probiotic bacteria is generically described *Bacillus.* No specific preferred *Bacillus* strains are mentioned and the working examples just refer to probiotic bacteria as such.

JP2000217567A describes a method to get high concentration of spores of Bacillus bacteria, and claims use of such Bacillus spores in fish foods. The description says that any Bacillus species may be used and as examples are listed *Bacillus pumils, Bacillus lentus, Bacillus laterosporus* and *Bacillus alvei.* All examples relate to result demonstrating the improved spore formation of the described method. No concrete data is provided in relation to a possible effect when uses as fish food.

JP2000103740A describes a medicine for fish and shellfishes which contains metabolites and products, such as a peptide obtained by the mixed culture of *Bacillus thuringiensis* and *Bacillus pumilus,* as an active principle. Based on specific examples is concluded that this mixed culture improves immunity activation and infection prevention for cultured fishes.

EP287699B1 (Calpis, Japan) describes that Bacillus subtilis C-3102 has a positive effect of increasing weight and improving feed efficiency, when it is given to fishes. Example 5 demonstrates an increasing weight gain effect for rainbow trout.

JP62138148A describes a feed additive for aquatic animals, containing microbial cells of *Bacillus cereus* or spores thereof as an active ingredient. It is said to have promoting effect on growth of fishes and preventing effect on bacterial diseases without causing problems of pollution of fish ground, resistant germs, etc.

RU2186576 describes a method of treatment and prevention of diseases in fish, including feeding to fish a curative fodder containing a probiotic agent, characterized in that used as the probiotic agent is *Bacillus subtilis* VKPM V-4759 in the form of spores) per one ton of fodder, over five days.

Chr. Hansen A/S (Denmark) commercializes a product with trade name BioPlus™2B. It comprises a mixture of *Bacillus licheniformis* and *Bacillus subtilis* spores. In various experiments, the positive effect of the addition of BioPlus2B has been demonstrated on average daily weight gain, feed consumption, feed conversion and/or mortality in piglets, sows, pigs for fattening, chickens for fattening, turkeys for fattening and calves.

In relation to fish there has not been described nor suggested any effects of BioPlus2B. Presently the mode of action of BioPlus2B in above-mentioned warm-blooded animals is poorly understood and it is well known to the skilled person that fish is an animal that in a number of ways is very different from above-mentioned warm-blooded animals.

### SUMMARY OF THE INVENTION

The problem to be solved by the present invention may be seen in the provision of an aquatic animal (e.g. fish) feed product capable of significantly increasing the resistance of aquacultured animal (e.g. fish) against relevant pathogenic microorganisms.

The solution is based on that the present inventors have identified that a fish feed product enriched with a probiotic *Bacillus licheniformis* bacteria is capable of significantly increasing the resistance of aquacultured fish against relevant pathogenic microorganisms. In working examples herein this is demonstrated for BioPlus2B (it comprises *Bacillus* *licheniformis* spores) enriched fish feed. It was demonstrated that such *Bacillus licheniformis* enriched fish feed product could significantly increase the relative survival rate of aquacultured fish when challenging the aquacultured fish by exposure to *Yersinia ruckeri. Yersinia* ruckeri is a pathogenic microorganism that is a causative agent for enteric redmouth disease.

Accordingly, a first aspect of the invention relates to an aquatic animal feed product comprising probiotic *Bacillus licheniformis* bacteria.

As said above, such a feed product is highly suitable for use as a feed product for an aquatic animal.

Accordingly, a second aspect of the invention relates to an aquaculture of aquatic animals wherein the aquatic animals are in contact with an aquatic animal feed product comprising a probiotic *Bacillus licheniformis* bacteria.

In a third aspect the invention relates to a method for feeding an aquatic animal present in an aquaculture comprising feeding the aquatic animal with an aquatic animal feed product comprising probiotic *Bacillus licheniformis* bacteria.

Without being limited to theory, it is believed that the probiotic *Bacillus licheniformis* bacteria are capable of significantly increasing the amount of aquatic animal antibodies against pathogenic microorganisms such as *Yersinia ruckeri.* This increase in antibodies then makes the aquatic animal more resistant to these pathogenic microorganisms. See working examples herein for further details. It is believed to be the first time that it is demonstrated that a probiotic organism gives its positive effect trough immune stimulation rather than by competitive exclusion.

A further advantage of a feed product comprising probiotic *Bacillus licheniformis* bacteria is that the growth weight gain and growth length of the aquatic animal is improved. See working examples herein for further details.

### DEFINITIONS

Prior to a discussion of the detailed embodiments of the invention is provided a definition of specific terms related to the main aspects of the invention.

The term "aquaculture" should be understood according to the art as an aquatic culture of aquatic animals wherein the animals are cultured in a physically defined space such as e.g. in cages.

The term "probiotic" is a well-defined term in the art and relates to a microorganism that when it has been in physical contact (e.g. when eaten) with an aquatic animal it confers health benefit to the animal.

The term *"Bacillus licheniformis"* is a well-known and well-defined term for this *Bacillus* bacteria specie. For further details see e.g. the standard reference book Bergeys Manual of Systematic Bacteriology. Based on his general knowledge, the skilled person is perfectly capable to determine whether or not a specific Bacillus bacterium of interest is a *Bacillus licheniformis* bacterium.

Embodiments of the present invention are described below, by way of examples only.

### DETAILED DESCRIPTION OF THE INVENTION

### Aquatic animal feed product

The aquatic animal feed product should comprise suitable aquatic animal feedstuff ingredients. The skilled person is aware of selecting the adequate ones in relation to the specific aquatic animal of interest. Herein, such suitable aquatic animal feedstuff ingredients may be termed aquatic animal feedstuff ingredients known per se.

These ingredients should preferably be in concentrations adjusted to meet animal's dietary requirements and may include nutrient ingredients such as animal protein products, at about 0-95 weight percent; plant protein products, at about 0-95 weight percent; poultry egg products, at about 0-25 weight percent.

Further, the aquatic animal feed product may also comprise other suitable ingredients such as Ergosan (an algine-based immunomodulator); antibiotics such as Sarafin, Romet, Terramycin at about 0.01-50 weight percent; cyanocobalamin at about 40-60 mg/kg; D-biotin at about 5-20 mg/kg; D-pantothenic acid at about 250-350 mg/kg; folic acid at about 10-30 mg/kg; L-ascorbyl-2-polyphosphate (STAY-C, stable form of vitamin C) at about 1,000-4,000 mg/kg; myo-inositol at about 3,000-4,000 mg/kg; niacin at about 600-800 mg/kg; p-amino-benzoic acid at about 350-450 mg/kg; pyridoxine hydrochloride at about 40-60 mg/kg; riboflavin at about 125-175 mg/kg; thiamine hydrochloride at about 50-80 mg/kg; choline chloride at about 6,500 7,500 mg/kg.

The aquatic animal feed product may be in any suitable form, such as a powder or in form of pellets or tablets.

The aquatic animal feed product may be in form of e.g. two different compositions one comprising the suitable aquatic animal feedstuff ingredients and the other comprising the probiotic *Bacillus* bacteria as described herein. In such a case the aquatic animal feed product is comprised of such two compositions and with suitable instructions to give them to the aquatic animal in conjunction either simultaneously or sequentially. In other words, while the aquatic animals are feed with the suitable aquatic animal feedstuff ingredients they should also be feed with the probiotic *Bacillus* bacteria as described herein.

Alternatively, the aquatic animal feed product may be in form of a composition comprising the suitable aquatic animal feedstuff ingredients and the probiotic *Bacillus* bacteria as described herein. This may e.g. be in the form of a suitable powder or in the form of pellets or tablets.

In order to e.g. improve some stability aspects of the probiotic bacteria it may be advantageous to provide the aquatic animal feed product as an stable emulsion of solids in-oil comprised of lipid soluble bioactive compounds such as inhibitory furanones dissolved in lipid forms of the continuous phase and with dry feed ingredients and the probiotic bacteria of interest forming the dispersed phase of the stable emulsion. See e.g. WO02/00035 for further details.

Further, the aquatic animal feed product may be in form of a capsule e.g. a microencapsulated product.

### Probiotic Bacillus bacteria

The probiotic *Bacillus licheniformis* bacteria may be any probiotic *Bacillus licheniformis* bacteria. Based on the herein disclosed information the skilled person is capable of selecting a specific *Bacillus licheniformis* of interest.

The product BioPlus™2B (Chr. Hansen A/S, Denmark) comprises the *Bacillus licheniformis* DSM5749 strain. As shown in working examples herein this *Bacillus licheniformis* DSM5749 strain is, in the present context, highly suitable and is therefore a preferred *Bacillus licheniformis* strain. This *Bacillus licheniformis* DSM5749 strain may herein also be termed CH 200.

Beside the probiotic *Bacillus licheniformis* bacteria the aquatic animal feed product may comprise any other microorganism of interest. Preferably, it is a probiotic microorganism of interest. Preferably, such a probiotic microorganism is a Gram negative bacterium or more preferably a Gram positive bacterium. A preferred Gram positive bacterium is a species selected from the group consisting of *Lactococcus* spp., *Lactobacillus* spp., Leu*conostoc spp., Oenococcus* spp., *Streptococcus* spp. and more preferably *Bacillus* spp.

Preferred other probiotic *Bacillus* bacteria is a *Bacillus* spp including but not limited to *Bacillus subtilis, Bacillus laterosporus, B. azotoformans, B. circulans, B. pumilus, B. lentus, B. alvei, B. thuringiensis, B. cereus* and *B. firmus.*

A most preferred other probiotic *Bacillus* specie is *Bacillus subtilis.* As illustrated in working examples herein a mixture of *Bacillus licheniformis* and *Bacillus subtilis* is, in the present context, highly suitable. These two *Bacillus* species have different biochemical characteristic leading to a possible synergy effect.

The product BioPlus™2B (Chr. Hansen A/S, Denmark) comprises the *Bacillus subtilis* DSM5750 strain. As shown in working examples herein this *Bacillus subtilis* DSM5750 strain is, in the present context, highly suitable and is therefore a preferred *Bacillus subtilis* strain. This *Bacillus subtilis* DSM5750 strain may herein also be termed CH 201.

Accordingly, in a preferred embodiment the aquatic animal feed product comprises a mixture of probiotic *Bacillus licheniformis* bacteria and probiotic *Bacillus subtilis* bacteria.

Preferably, the aquatic animal feed product comprises the probiotic *Bacillus licheniformis* bacteria in a concentration of at least 10 E6 CFU/g feed, more preferably in a concentration of at least 10 E7 CFU/g feed, even more preferably in a concentration of at least 10 E8 CFU/g feed. Generally, the aquatic animal feed product comprises the probiotic *Bacillus licheniformis* bacteria in a concentration of lees than 10 E14 CFU/g feed.

When the aquatic animal feed product comprises a mixture of the probiotic *Bacillus licheniformis* bacteria and one or more another probiotic microorganisms (such as *Bacillus subtilis)* it is preferred that there are, based on total amount of probiotic microorganisms, from 5 to 95% of the probiotic *Bacillus licheniformis* bacteria and from 5 to 95% of the other probiotic microorganism. The skilled person does of coerce understand that the total sum cannot exceed 100%. In other words, if 80% is *Bacillus licheniformis* then the other microorganisms cannot exceed 20%.

When another probiotic microorganism is *Bacillus subtilis,* it is preferred that there are, based on total amount of probiotic microorganisms, from 35 to 65% of the probiotic Ba*cillus licheniformis* bacteria and from 35 to 65% of the *Bacillus subtilis* bacteria.

The probiotic *Bacillus* bacteria may e.g. be in the form of bacterial spores, vegetative bacterial cells, bacterial cell walls or any combination thereof. Preferably probiotic *Bacillus* bacteria are in the form of vegetative bacterial cells or more preferably in the form of bacterial spores.

Preferably, the probiotic *Bacillus* bacteria are included in the aquatic animal feed product as dried (preferably freeze-dried) bacteria. However, they may also be included as a frozen culture of bacteria.

### Aquatic animal

It is appreciated that the aquatic animal feed product as described herein is particularly useful within the fishing and aquaculture industries, primarily by causing a reduced viability of microbial organisms which exert harmful effects on shellfish, cartilaginous fish, fin fish or aquatic mammals.

Shellfish may comprise the group of filter-feeding bivalves such as e.g. clams, oysters, scallops and mussels and may in addition comprise lobsters, crabs and shrimps.

Finfish may be selected from the group consisting of but are not limited to the salmonid species including Atlantic salmon *(Salmo salar),* rainbow trout *(Oncorhynchus mykiss).*

Further preferred aquatic animal is a fish selected from the group consisting of a gadid species including *Gadus callarias,* sea trout *(Salmo trutta)* and sea bass *(Dicentrarchus labrax)* and cod, eel as well as fresh water finfish and carp. Further, an aquatic animal may be a dolphin or a whale.

Preferably the aquatic animal is an animal farmed in an aquaculture. The aquatic animal may be in an early developmental stage e.g. larvae and juvenile animals or be in a later developmental stage than juvenile stage.

### Resistance against pathogenic microorganisms

As said above the aquatic animal feed product, as described herein, is capable of significantly increasing the resistance of aquacultured animals against relevant pathogenic microorganisms. In working examples herein this is demonstrated for BioPlus2B (it comprises *Bacillus licheniformis* spores) enriched fish feed. It was demonstrated that such *Bacillus licheniformis* enriched fish feed product could significantly increase the relative survival rate of aquacultured fish when challenging the aquacultured fish by exposure to *Yersinia ruckeri. Yersinia ruckeri* is an important pathogenic microorganism that is a causative agent for enteric redmouth disease.

In working examples herein it is shown that for aquacultured rainbow trout an aquatic animal feed product, as described herein, could increase the relative survival rate of the rainbow trout against *Yersinia ruckeri* by at least 20% as compared to a control group feed with the corresponding animal feed product without the probiotic *Bacillus* bacteria.

In the present context, the fish rainbow trout is believed to be a representative aquatic animal. In other words, when the positive resistance effect against *Yersinia ruckeri* has been demonstrated for rainbow trout it also applies to other aquatic animal in general. Without being limited to theory, it is presently believed to be the first time that it has been demonstrated that a probiotic microorganism may give such a positive effect against an important pathogenic microorganism such as *Yersinia ruckeri.*

Accordingly, a preferred embodiment relates to an aquatic animal feed product, as described herein, characterized by that when it is used for feeding an aquaculture of rainbow trout it is capable of providing an at least 2% increase in relative survival rate of the rainbow trout as compared to a control group feed with the corresponding animal feed product without the probiotic *Bacillus licheniformis* bacteria when challenging the rainbow trout with *Yersinia ruckeri.*

As said above, in a preferred embodiment the aquatic animal feed product comprises both probiotic *Bacillus licheniformis* bacteria and probiotic *Bacillus subtilis* bacteria, since these two *Bacillus* species have different biochemical characteristic leading to a possible synergy effect. In this special situation in relation to this specific embodiment the corresponding animal feed control product should be without both the probiotic *Bacillus licheniformis* bacteria and the probiotic *Bacillus subtilis* bacteria.

In working examples herein are provided a suitable test assay to evaluate the increased resistance of rainbow trout against challenge with *Yersinia ruckeri.* In the present context this is a preferred assay. Based on the relevant information of the working examples, the skilled person may, based on his general knowledge, made a corresponding assay that would be sufficient accurate to, within normal measurement uncertainty, determine the increase in relative survival rate of the rainbow trout.

In short, this assay comprises the steps of:
(i) make two populations of an aquaculture of rainbow trout consisting of 100 4 month old fish
(ii) feed one population, in an 1m x 1 m x 1m aquarium, with the aquatic animal feed product as described herein (28 days with a daily dose of three percent of their biomass) and the other population similarly but using a corresponding animal feed control product without the probiotic *Bacillus licheniformis* bacteria (control group)
(iii) after the 28 days of feeding 10 fish of each group after taken out and transported over to new aquariums
(iv) after two days of acclimatization the fish are challenged with *Yersinia ruckeri* by intra peritoneal injection of a dose of about 2,5 E6 CFU per fish
(v) observe the fish during a period of 28 days in separate aquariums (12-13°C) and calculate daily the mortality and relative percent survival.

Preferably the aquatic animal feed product, as described herein, is characterized by that when it is used for feeding an aquaculture of rainbow trout it is capable of providing an at least 5% (more preferably at least 10%, even more preferably at least 15%) increase in relative survival rate of the rainbow trout as compared to a control group feed with the corresponding animal feed product without the probiotic *Bacillus licheniformis* bacteria when challenging the rainbow trout with *Yersinia ruckeri.*

### An aquaculture of aquatic animals wherein the aquatic animals are in contact with the aquatic animal feed product as described herein

Preferably this relates to an industrial relevant aquaculture wherein the aquatic animals are cultured in a physically defined space of at least 1m x 1m x 1m such as e.g. in an aquarium of at least 1m x 1m x 1m. The physically defined space of the aquaculture may be significantly bigger such e.g. as 10m x 10m x 5 m.

The term "the aquatic animals are in contact with the aquatic animal feed product" denotes a typical situation just after feeding where the aquatic animals are in contact with the aquatic animal feed product, for instance while they are eating the feed product.

Feeding may be done according to the art and the skilled person is aware of how to properly feed aquatic animals present in an aquaculture.

In a preferred embodiment the aquatic animals of the aquaculture are in contact with an amount of aquatic animal feed product that corresponds to from 0.5% to 25 % of the total biomass of the aquatic animals of the aquaculture, more preferably the aquatic animals of the aquaculture are in contact with an amount of aquatic animal feed product that corresponds to from 1% to 10% of the total biomass of the aquatic animals of the aquaculture.

### A method for feeding an aquatic animal present in an aquaculture comprising feeding the aquatic animal with an aquatic animal feed product as described herein

As said above feeding may be done according to the art and the skilled person is aware of how to properly feed aquatic animals present in an aquaculture.

Preferably, the aquatic animals of the aquaculture are feed with an amount of aquatic animal feed product that corresponds to from 0.5% to 25% of the total biomass of the aquatic animals of the aquaculture, more preferably with an amount of aquatic animal feed product that corresponds to from 1% to 10% of the total biomass of the aquatic animals of the aquaculture. Preferably, the aquatic animals are feed with a daily dose of the aquatic animal feed product.

As illustrated in e.g. working examples herein the aquatic animal feed product, as described herein, also improves the weight gain and/or feed conversion.

Accordingly, a preferred embodiment relates to a method for feeding an aquatic animal present in an aquaculture comprising feeding the aquatic animal with an aquatic animal feed product, as described herein, wherein the weight gain is at least 2% such as at least 5%, 7%, 10%, 15%, 30%, 40%, 50% or even more as compared to a control group feed with the corresponding animal feed product without the probiotic *Bacillus licheniformis* bacteria.

Further, a preferred embodiment relates to a method for feeding an aquatic animal present in an aquaculture comprising feeding the aquatic animal with an aquatic animal feed product, as described herein, wherein feed conversion is increased by at least 2% such as at least 4%, 7%, 10%, 15%, 20%, 30% or even more as compared to a control group feed with the corresponding animal feed product without the probiotic *Bacillus licheniformis* bacteria.

Even further, a preferred embodiment relates to a method for feeding an aquatic animal present in an aquaculture comprising feeding the aquatic animal with an aquatic animal feed product, as described herein, wherein there is obtained an at least 2% increase in relative survival rate of the aquatic animal as compared to a control group feed with the corresponding animal feed product without the probiotic *Bacillus licheniformis* bacteria when challenging the aquatic animal with *Yersinia ruckeri.*

As said above, in a preferred embodiment the aquatic animal feed product comprises both probiotic *Bacillus licheniformis* bacteria and probiotic *Bacillus subtilis* bacteria, since these two *Bacillus* species have different biochemical characteristic leading to a possible synergy effect. In this special situation in relation to this specific embodiment the corresponding animal feed control product should be without both the probiotic *Bacillus licheniformis* bacteria and the probiotic *Bacillus subtilis* bacteria.

As explained in section "Resistance against pathogenic microorganisms" above, in working examples herein are provided a suitable test assay to evaluate the increased resistance against challenge with *Yersinia ruckeri.* In the section above was focused on rainbow trout. However, based on this section and the further information herein the skilled person may, based on his general knowledge, made a corresponding assay for any aquatic animal of interest that would be sufficient accurate to, within normal measurement uncertainty, determine the increase in relative survival rate of the aquatic animal of interest.

Preferably, in the method for feeding an aquatic animal, as described herein, there is obtained an at least 5% (more preferably at least 10%, even more preferably at least 15%) increase in relative survival rate of the aquatic animal as compared to a control group feed with the corresponding animal feed product without the probiotic *Bacillus licheniformis* bacteria when challenging the aquatic animal with *Yersinia ruckeri.*

### A separate aspect of the invention

As said above it is presently believed to be the first time that it has been demonstrated that a probiotic microorganism may give the herein described positive effect against an important pathogenic microorganism such as *Yersinia ruckeri.*

Without being limited to theory, it is believed that based on the information provided herein the skilled person is able to, based on his common general knowledge, to identify other probiotic microorganisms and combinations of these that also could give this positive effect.

Accordingly, a separate aspect of the invention relates to a method for feeding an aquatic animal present in an aquaculture comprising feeding the aquatic animal with an aquatic animal feed product comprising a probiotic microorganism wherein there is obtained an at least 2% increase in relative survival rate of the aquatic animal as compared to a control group feed with the corresponding animal feed product without the probiotic microorganism when challenging the aquatic animal with *Yersinia ruckeri.*

Preferably, the probiotic microorganism is a Gram negative bacterium or more preferably a Gram positive bacterium. A preferred Gram positive bacterium is a species selected from the group consisting of *Lactococcus* spp., *Lactobacillus* spp., *Leuconostoc spp., Oenococcus* spp., *Streptococcus* spp. and more preferably *Bacillus* spp.

All other aspects, embodiments and other information herein are also relevant and included in relation to this separate aspect of the invention.

### EXAMPLES

### Material and methods

### Fish

The used rainbow trout was obtained by hatching out eggs provided to Borhholms Lakseklækkeri, Denmark from Faarup Mølle Dambrug, Denmark. The eggs were sterilized at arrival and were hatched out in re-circulated fresh water at 7°C.

Rainbow trout fish fry was hatched out the 1 of Marts 2002 and was thereafter kept in 10°C cold water in special trays and after that kept in aquariums of 1m x 1m x 1m. They were feed with commercial rainbow trout feed (BioMar, Denmark) until they were used in the experiments.

### The aquaculture site

The aquaculture site was at Borhholms Lakseklækkeri, Denmark. The water at the site was re-circulated fresh water and was continuously kept free of pathogens. The water was circulated by mechanical and biologic filtration and UV-treatment.

### Experimental design

4 month old rainbow trout fish were placed in 6 groups each of 100 individual fish. They were cultured in aquariums of 1m x 1m x 1m in re-circulated water at 10°C.

Two groups were control groups that were feed with BioMar rainbow trout feed without addition of probiotic.

Two groups were feed with the same feed enriched with the algine-based immunomodulator product Aquavac Ergosan (Aquaculture Vaccines Ltd.). It was added to 0.2% in mixed feed according to manufactures instructions.

Two groups were feed with the same feed enriched with probiotic *Bacillus* bacteria. The food was enriched with BioPlus2B (Chr. Hansen A/S). The quantitative composition of BioPlus 2B was as follows:

| | |
|---|---|
| Bacillus licheniformis CH 200 spore concentrate | 0.5% |
| Bacillus subtilis CH 201 spore concentrate | 0.5% |
| Sodium alumino silicate (E 554) | 1.0% |
| Whey permeate | 98.0% |

BioPlus2B was added to get a Bacillus concentration of 4 E9 CFU/g feed.

All fish were given a daily feed portion that corresponded to 3% of their total biomass. The experiment was started the 2 July 2002 and finished 13 August 2002. Samples were taken out and analyzed 2 July 2002, 16 July 2002, 30 July 2002 and 13 August 2002.

### Samples analyses

### Blood

At each sample analyze there was taken a blood sample of 10 fish from each group with the exception of the first sample outtake of naive fish, where there were taken blood samples from 20 fish in total. The blood sample test was performed in heparinsed heamatocrit tubes (10 or 20 microliter volume). The heamatocrti analyze was done after 5 minutes centrifugation at 1000 x g. After that the tubes was cut, volume calculated and the plasma sample was kept at -20°C until further analyzes. Further, a blood cell plating was done on object glasses, which were air dried and thereafter fixed in absolute ethanol. The cells were colored with Giemsa and the number of lymphocytes per 1000 blood cells was counted by microscope. The plasma sample was also used for protein detection, where a BCA-kit (Pierce) was used.

### Challenge

At each day where samples were taken out there were collected 10 fish from each group. These fish were transported alive to other aquariums. After a 2 days acclimatization period the fish where exposed to *Yersinia ruckeri* by intra peritoneal injection of a dose of about 2.5 E6 CFU per fish. In separate aquariums (12-13°C), the fishes were continuously observed for a period of 28 thereafter. The mortality was daily registered and the relative percent survival was calculated.

### Antibody determination

In order to determine if there were different antibody profiles against *Yersinia ruckeri* in the different groups of rainbow trout samples the plasma samples were also used in an ELISA test. Microtiter plates (96 wells immunoplates) were coated with formalin killed *Yersinia ruckeri* (200 microliter per well, 1x10⁵ cfu/ml) at standard conditions. The assay was done by standard methods of blocking and washing. Plasma from the test sample fishes (diluted 1:100) was used as primary antibody, rabbit antibody that reacted with the rainbow trout antibody was used as secondary antibody and as tertiary antibody was used a peroxidase conjugated goat antibody (Sigma). Chromogenic substrate was OPD (Sigma) and the absorbance was measured at 492 nm.

### Bacillus spores in feed and fish intestinal tract

At day 42 there was taken samples from the different test groups in order to analyze for presence of *Bacillus subtilis* and *Bacillus licheniformis* in the intestinal tract of the fishes. Further there were taken samples of the used BioMar feed.

### Length and weight gain

At each day and time where fish samples were taken out the length and the weight of the fished were determined.

### Results

### General

There was not registered any anorexia or other bad state in any of the test groups. The rainbow trouts ate all the feed in a satisfactory manner.

### Challenge

In the *Yersinia ruckeri* challenge experiments did the Ergosan group not have any improved survival rate in relation to the control group after 14 and 28 days. A weak limited improved survival rate was seen after 42 days of feeding with the Ergosan enriched feed. Fishes that were feed with BioPlus2B enriched feed had a significant improved survival rate at all tests. The relative survival rate was 20%, 20%, 35% after respectively 14, 28 and 42 days feeding with the BioPlus2B enriched feed.

### Blood parameters

In relation to plasma protein, haematocrit values and lymphocyte cell numbers it were not possible to identify significant measurable differences between the individual test groups. However, in relation to antibody profiles against *Yersinia ruckeri* it was clearly seen that there were a significant higher *Yersinia ruckeri* antibody titer in the BioPlus2B enriched feed group as compared to the Ergosan enriched feed group.

### Bacillus spores in feed and fish intestinal tract

Analyses demonstrated a high concentration of the two Bacillus species in the fishes feed with BioPlus2B enriched feed plus in the BioPlus2B enriched feed as such.

### Length and weight gain

Both the length and weight gain was significantly increased in the BioPlus2B group as compared to both the control and Ergosan group.

### Discussion

This investigation demonstrated that BioPlus2B enriched fish feed product could significantly increase the relative survival rate of aqua-cultured rainbow trout fish when challenging the aqua-cultured fish by exposure to the pathogenic *Yersinia ruckeri.*

The date demonstrated that there were a significant higher *Yersinia ruckeri* antibody titer in the BioPlus2B enriched feed group as compared to the Ergosan enriched feed group. It is believed this increase in antibodies then makes the rainbow trout more resistant to the pathogenic *Yersinia ruckeri.*

Further, both the length and weight gain was significantly increased in the BioPlus2B group as compared to both the control and Ergosan group.

### REFERENCES

WO 02/00035
JP 2000217567A
JP 2000103740A
EP 287699B1 (Calpis, Japan)
JP 62138148 A
RU 2186576
BioPlus™2B (Chr. Hansen A/S, Denmark)

## Claims

1. An aquatic animal feed product comprising a probiotic *Bacillus licheniformis* bacteria.

2. The aquatic animal feed product of claim 1, wherein the product comprises aquatic animal feedstuff ingredients known per se.

3. The aquatic animal feed product of claims 1 or 2, wherein the aquatic animal feed product comprises the probiotic *Bacillus licheniformis* bacteria in a concentration of at least 10 E6 CFU/g feed.

4. The aquatic animal feed product of any of claims 1 to 3, wherein the product comprises a mixture of probiotic *Bacillus licheniformis* bacteria and probiotic *Bacillus subtilis* bacteria.

5. The aquatic animal feed product of any of claims 1 to 4, **characterized by** that when it is used for feeding an aquaculture of rainbow trout it is capable of providing an at least 2% increase in relative survival rate of the rainbow trout as compared to a control group feed with the corresponding animal feed product without the probiotic *Bacillus licheniformis* bacteria when challenging the rainbow trout with pathogenic *Yersinia ruckeri.*

6. An aquaculture of aquatic animals wherein the aquatic animals are in contact with an aquatic animal feed product comprising a probiotic *Bacillus licheniformis* bacteria of any of claims 1 to 5.

7. The aquaculture of an aquatic animals of claim 6, wherein aquatic animals of the aquaculture are in contact with an amount of aquatic animal feed product that corresponds to from 0.5% to 10% of the total biomass of the aquatic animals of the aquaculture.

8. The aquaculture of aquatic animals of claims 6 or 7, wherein the aquatic animals are finfish such as salmonid species including Atlantic salmon *(Salmo salar)* and rainbow trout *(Oncorhynchus mykiss).*

9. A method for feeding an aquatic animal present in an aquaculture comprising feeding the aquatic animal with an aquatic animal feed product comprising a probiotic *Bacillus licheniformis* bacteria of any of claims 1 to 5.

10. The method for feeding an aquatic animal of claim 9, wherein the aquatic animal is a finfish such as a salmonid specie including Atlantic salmon *(Salmo salar)* and rainbow trout *(Oncorhynchus mykiss).*
